# EUROPEAN PATENT SPECIFICATION

(11) **EP 1 725 217 B1**
(45) Date of publication and mention of the grant of the patent: **06.08.2008**
(21) Application number: 05708710.8
(22) Date of filing: 10.03.2005
(51) Int. Cl.: A61K 9/14, A61K 9/20, A61K 31/522, C07D 473/18

(54) **PROCESSES FOR THE PREPARATION OF SOLID DOSAGE FORMS OF AMORPHOUS VALGANCICLOVIR HYDROCHLORIDE**
VERFAHREN ZUR HERSTELLUNG VON FESTEN DOSIERFORMEN VON AMORPHEM VALGANCICLOVIR-HYDROCHLORID
PROCEDES POUR LA PREPARATION DE FORMES POSOLOGIQUES SOLIDES DE CHLORHYDRATE DE VALGANCICLOVIR AMORPHE

(30) Priority: 10.03.2004 IN DE04102004
(43) Date of publication of application: 29.11.2006
(73) Proprietor: RANBAXY LABORATORIES, LTD., Gurgaon 122 001 HR (IN)
(72) Inventor: SINGH, Romi, Barat, Varanasi 221002, Uttar Pradesh (IN); NAGAPRASAD, Vishnubhotla, Hyderabad 500072, Andhra Pradesh (IN); SINGH, Nidhi, New Delhi 110044, Delhi (IN)
(74) Representative: Cronin, Brian Harold John
(86) International application number: PCT/IB2005/000615
(87) International publication number: WO 2005/087198

(56) References cited:
- WO-A-20/04010998
- US-A1- 2004 062 805
- US-B1- 6 248 363
- US-B1- 6 395 029

## Description

### Field of the Invention

The present invention relates to a process for the preparation of solid dosage forms of amorphous valganciclovir hydrochloride by dry method.

### Background of the Invention

Valganciclovir hydrochloride, a prodrug of gancilcovir, is used in the treatment of cytomegalovirus (CMV) retinitis in patients with acquired immunodeficiency syndrome (AIDS) and for the prevention of CMV disease in kidney, heart and for kidney-pancrease transplant patients who are at high risk. Valganciclovir hydrochloride is hydrochloride salt of the L-valyl ester of ganciclovir. Chemically, it is L-Valine, 2-[(2-amino-1,6-dihydro-6-oxo-9H-purin-9-yl)methoxy]-3-hydroxypropyl ester, monohydrochloride. It is available as tablets under the brand name Valcyte® containing crystalline valganciclovir hydrochloride.

U.S. Patent No. 6,083,953 discloses valganciclovir hydrochloride and processes for its preparation. Therein it is set forth that valganciclovir hydrochloride was developed with a view to improve the bioavailability of gancilcovir, which has a poor bioavailability when administered orally. The higher oral doses of ganciclovir are required to achieve a therapeutic concentration in the blood. Valganciclovir hydrochloride when administered orally is reported to have better bioavailability than ganciclovir given orally.

It is known that the amorphous form of a drug may have certain advantages over the crystalline form, for example, amorphous form can be more soluble or can have a higher rate of solubility in water than the crystalline form and consequently the drug may show improved bioavailability, for example, due to the faster dissolution of the drug in the gastrointestinal fluid. It is with this view that we have prepared dosage forms comprising valganciclovir hydrochloride in which valganciclovir hydrochloride is present in the amorphous form. Without being bound by any theory, these dosage forms may further improve the oral bioavailability of valganciclovir and ultimately that of ganciclovir.

However, it has been found that amorphous valganciclovir hydrochloride as such is very fine and fluffy material, with relatively low bulk and tap density. This property can make it difficult to formulate into a dosage form with uniformity of weight, hardness, and other desirable tablet properties. Wet granulation needs to be avoided, as addition of a solvent along with subsequent removal in way of drying the granules at elevated temperatures may convert the amorphous form to crystalline form.

The direct compression technique may not be desirable for a drug with a bulk density less than 0.2 g/cc, due to poor flow of the material leading to non-uniform die-fill and subsequent weight variation. However, direct compression could be a method of choice when the amorphous valganciclovir hydrochloride is so processed that it has a bulk density of at least 0.2 g/cc or more. This can be achieved during chemical manufacturing by variation in the processing parameters or by a physical process of compaction and milling.

There is a need for simple method of production, which does not require wet granulation with organic solvents or water and do not require an additional step of drying.

### Summary of the Invention

We hereby report processes for the preparation of solid dosage forms comprising amorphous valganciclovir hydrochloride by a dry process which may be, for example, dry granulation or direct compression. The processes give dosage forms with uniformity of weight, sufficient hardness and friability.

In one general aspect, the present disclosure relates to dry processes for the preparation of solid dosage forms comprising amorphous valganciclovir hydrochloride and one or more pharmaceutically acceptable excipient(s).

In another general aspect, herein are provided processes for the preparation of solid dosage forms of amorphous valganciclovir hydrochloride wherein the processes comprise mixing amorphous valganciclovir hydrochloride with one or more pharmaceutically acceptable excipient(s) and forming into solid dosage forms.

In another general aspect, herein are provided processes for the preparation of solid dosage forms of amorphous valganciclovir hydrochloride wherein the processes comprise mixing amorphous valganciclovir hydrochloride with one or more pharmaceutically acceptable excipient(s) and compressing into a tablet.

In another general aspect, herein are provided processes for the preparation of solid dosage forms of amorphous valganciclovir hydrochloride wherein the processes comprise compacting valgancclovir hydrochloride alone or mixed with one or more pharmaceutically acceptable excipient(s) by, for example, roller compaction or slugging; sizing the compacts into granules by milling; optionally mixing the granules with one or more of pharmaceutically acceptable excipients and forming a solid dosage form.

In another general aspect, herein are provided processes for the preparation of solid dosage forms of amorphous valganciclovir hydrochloride wherein the processes comprise compacting valganciclovir hydrochloride alone or mixed with one or more pharmaceutically acceptable excipient(s) by, for example, roller compaction or slugging; sizing the compacts into granules by milling; optionally mixing the granules with one or more of pharmaceutically acceptable excipients and compressing into a tablet.

In another general aspect, there is provided a pharmaceutical composition for the treatment of cytomegalovirus (CMV) retinitis in patients with acquired immunodeficiency syndrome (AIDS) and for the prevention of CMV disease in kidney, heart and for kidney-pancrease transplant patients, comprising a solid dosage form of amorphous valganciclovir hydrochloride.

### Brief Description of the Drawings

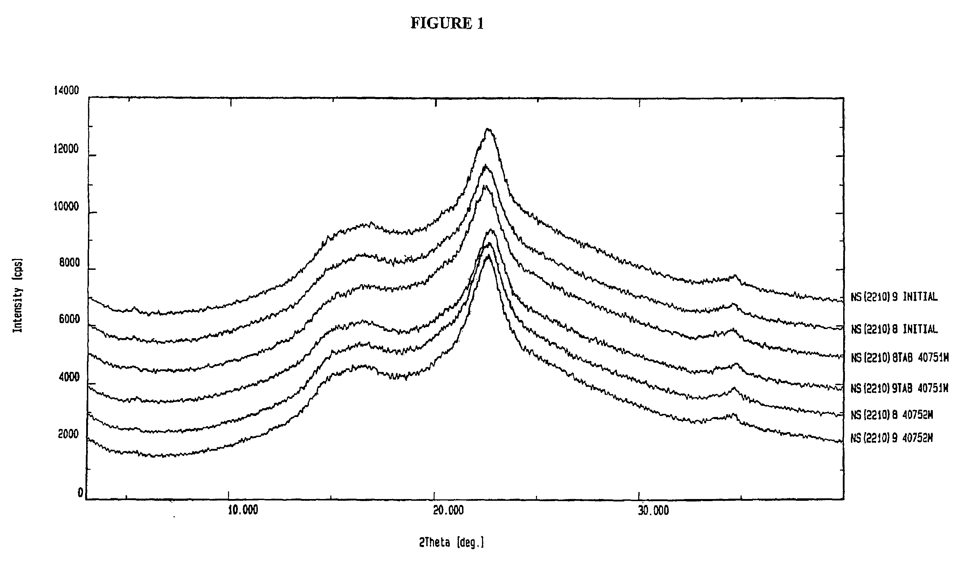

Fig. 1 is a set of x-ray diffractogram of tablets of Example 1 recorded upon production and after storage for two months at 40° C and 75% relative humidity.

### Detailed Description of the Invention

The present invention provides solid dosage forms comprising valganciclovir hydrochloride in amorphous form prepared by a dry process. The process is simple and economical as it does not require any solvents, as in the case of wet granulation processes, the most commonly followed process, which requires additional steps of drying the granules. The amorphous form resists conversion to a crystalline form by following the dry processes as described herein.

The term "solid dosage form" as used herein includes granules, tablets or coated tablets and capsules filled with granules or tablets prepared as per the embodiments described herein. Particularly suitable solid dosage forms are tablets.

The amorphous valganciclovir hydrochloride in the solid dosage form is present in a therapeutically effective amount. Typically, the drug may comprise from about 1mg to about 1000 mg, for example, from about 50 mg to about 800 mg. The amorphous valganiclovir can be prepared by methods described in Indian Patent Application No.1052/DEL/2003 filed 28 August 2003.

Additionally, other drugs in a therapeutically effective amount can also be combined with the present dosage forms.

The pharmaceutically acceptable excipients are those known to the skilled in the art and may be selected from fillers, binders, disintegrants, glidant and lubricants. These excipients may be present intragranularly or extragranularly or both.

The fillers may be selected from microcrystalline cellulose, mannitol, sucrose, lactose, dextrose, calcium carbonate, sorbitol and the like. The filler may be present in an amount of about 15% to about 60%, for example, from about 20% to 40% by weight of the dosage form.

The binders may be selected from polyvinylpyrrolidone, hydroxypropylcellulose, hydroxypropylmethylcellulose, starch and starch based binders, gelatin, gums and the like. The binder may be present in an amount of about 0.1% to about 10%, for example, from about 1% to about 5% by weight of the dosage form.

The disintegrant may be selected from crospovidone, croscarmellose sodium, starch, hydroxypropylcellulose, hydroxypropylmethylcellulose, gums, sodium starch glycolate and the like. The disintegrant may be present in an amount of about 1% to about 40%, for example, from about 2% to about 20% by weight of the dosage form.

The lubricants and glidants may be selected from talc, colloidal silicon dioxide, magnesium stearate, stearic acid and sodium stearyl fumarate. These may be present in an amount of about 0.1% to about 2% by weight of the dosage form.

The compaction of the drug or the mixture comprising the drug and excipient(s) into compacts may be carried out by slugging or by roller compaction, particularly suitable is roller compaction.

The roller compactor functions by uniformly applying pressure on a mixed powder blend by passing the blend between two counter-rotating rollers. The pressure imparted on the blend by the rollers compresses the powder into a compact, such as a sheet or ribbon, which is typically milled to produce granules.

In one general aspect of the process, the valganciclovir hydrochloride can be mixed with one or more of pharmaceutical excipients such as filler, binder, disintegrant and lubricant described above in a suitable blender. The mixing time can vary from about 10 to 60 minutes. The resultant blend can either be directly compressed into solid dosage form or compacted by roller compaction.

The resultant blend for compaction is subsequently transferred to a roller compactor in a known manner. The roller speed, roller gap width and force of compaction are then adjusted and the blend is fed through the roller compactor. The typical force and other conditions can be easily selected and adjusted by those skilled in the art. For example, the compaction pressure may be between 25 to 75 bar, for example, between 35 to 55 bar. For maintaining the steady output of the compact material from the roller compactor, the rollers may be rotated at a speed of between 1 to 20 rpm, for example, between 2 to 10 rpm, or between 3 to 5 rpm. When in contact with the counter rotating rollers of the roller compactor, the compression force imparted on the blend by rollers converts the powdered form into a ribbon or compaction sheet. This compact sheet is fed to a mill, such as an oscillating mill, fitted with a screen. The screen can be selected with variable hole diameters depending upon the size of the granules required. After passing through the mill and the screen, the compact is converted into granules of the desired particle size distribution.

The granules obtained as above may be filled into capsules or packed in a sachet. The granules can also be mixed with one or more of pharmaceutically acceptable excipients and compressed into tablets.

As an alternative to roller compaction, slugging may be used for preparing solid dosage forms such as a tablet. The process is simple, low cost and effective. Slugging may be carried out by means of a tablet press. The drug either alone or mixed with other excipients is precompressed on a heavy duty press. The slug so formed is milled into granules and recompressed into tablet. The granules may also optionally be mixed with other extragranular excipients prior to compression into a tablet.

Both the processes, i.e., roller compaction and slugging generate fines during the milling step. A portion of these fmes can be mixed with granules and compressed into a tablet or can be easily recycled by collecting them and again compacted.

The tablet must be of sufficient hardness to withstand the packaging, transport or coating process without chipping or breaking. The hardness of the tablets can be measured by known methods. The hardness should not be so high that it adversely affects disintegration and dissolution rates of the tablets. Preferably, the hardness of these amorphous valganciclovir hydrochloride tablets is about 10 kP to about 30 kP, for example, about 15 kP to about 25 kP.

Another measure of durability of the tablet is the test for friability. A friability values of about 1% is acceptable, but friability below about 0.8% is particularly suitable. The tablets prepared as per the process can have friability of less than 0.8%, for example, less than 0.5%.

In one embodiment, processes provided herein comprise mixing amorphous valganciclovir hydrochloride with one or of pharmaceutically acceptable excipients and compressing the blend into a tablet.

In one embodiment, processes provided herein comprise compacting amorphous valganciclovir hydrochloride alone or mixed with a lubricant using roller compactor; milling and sizing the compacts into granules with a desired particle size distribution; mixing with extragranular pharmaceutically acceptable excipient(s) and compressing into a tablet using appropriate tooling.

In another embodiment, processes herein comprise mixing amorphous valganciclovir hydrochloride, filler, binder, disintegrant and lubricant and compacting the mixture using roller compactor; milling and sizing the compacts into granules with a desired particle size distribution; mixing with lubricant and compressed into a tablet using appropriate tooling.

In still another embodiment, processes provided herein comprise mixing amorphous valganciclovir hydrochloride, filler, binder, disintegrant and lubricant and compacting the mixture using roller compactor; milling and sizing the compacts into granules with a desired particle size distribution; mixing with one or more of filler, binder, disintegrant and lubricant and compressed into a tablet using appropriate tooling.

In yet another embodiment, processes provided herein comprise compacting amorphous valganciclovir hydrochloride alone or mixed with one or more of filler, binder, disintegrant and lubricant, milling and sizing the compacts into granules with a desired particle size distribution and filling into a capsule dosage form.

In still another embodiment, processes provided herein comprise compacting amorphous valganciclovir hydrochloride alone or mixed with one or of pharmaceutically acceptable excipients by slugging; milling and sizing the slugs into granules with a desired particle size distribution; optionally mixing the granules with one or more of filler, binder, disintegrant and lubricant and compressed into a tablet.

When the solid dosage form is a tablet then it may additionally be coated with coating compositions like Opadry® AMB (with or without a non aqueous subcoat) sold by Colorcon to impart moisture protection on stability. Such a coating may comprise about 3 - 10%w/w of the tablet. The tablet may also be coated with coating compositions like Opadry® or Lustreclear® sold by Colorcon using non-aqueous or aqueous systems, preferably non-aqueous system to impart aesthetic appeal as well as a barrier to the external environment. Such a coating may comprise about 3-10%w/w of the tablet.

The invention described herein can further be illustrated by the following examples but these do not limit the scope of the invention.

### EXAMPLE 1

| **Ingredients** | **Quantity (mg)** |
|---|---|
| Valganciclovir hydrochloride (amorphous) eq. to 450 mg of valganciclovir | 496.3 |
| Microcrystalline cellulose | 159.95 |
| Cross-linked polyvinylpyrrolidone | 21.0 |
| Polyvinylpyrrolidone | 14.0 |
| Magnesium stearate | 8.75 |
| **Total** | **700** |

### Procedure:

Valganciclovir hydrochloride (amorphous) was mixed in a blender with microcrystalline cellulose, cross-linked polyvinylpyrrolidone, polyvinylpyrrolidone and magnesium stearate and compressed into tablet using appropriate tooling.

### EXAMPLE 2

| **Ingredients** | **Quantity (mg)** |
|---|---|
| **Intragranular** | |
| Valganciclovir hydrochloride (amorphous) eq. to 450 mg of Valganciclovir | 496.3 |
| Microcrystalline cellulose | 159.95 |
| Cross-linked polyvinylpyrrolidone | 21.0 |
| Polyvinylpyrrolidone | 14.0 |
| Magnesium stearate | 3.50 |

| **Extragranular** | |
|---|---|
| Magnesium stearate | 5.25 |
| **Total** | **700** |

### Procedure:

Valganciclovir hydrochloride (amorphous) was mixed with microcrystalline cellulose, cross-linked polyvinylpyrrolidone, polyvinylpyrrolidone and magnesium stearate and compacted with a roller compactor. The compacts were sized into granules by milling, mixed with magnesium stearate and compressed into tablet using appropriate tooling.

The tablets of Example 1 were subjected to accelerated stability testing. The tablets were kept at 40°C and 75% relative humidity for two months. The XRD data at the end of the two months period showed no change in amorphous nature in comparison to the initial scan of the amorphous valganciclovir hydrochloride, as shown in Fig. 1.

### EXAMPLE 3

| **Ingredients** | **Quantity (mg)** |
|---|---|
| **Intragranular** | |
| Valganciclovir hydrochloride (amorphous) eq. to 450 mg of Valganciclovir | 496.3 |
| Magnesium stearate | 3.50 |

| **Extragranular** | |
|---|---|
| Microcrystalline cellulose | 159.95 |
| Cross-linked polyvinylpyrrolidone | 21.0 |
| Polyvinylpyrrolidone | 14.0 |
| Magnesium stearate | 5.25 |
| **Total** | **700** |

### Procedure:

Valganciclovir hydrochloride (amorphous) and magnesium stearate were mixed in a blender and compacted using a roller compactor. The compacts were sized into granules by milling, mixed with microcrystalline cellulose, cross-linked polyvinylpyrrolidone, polyvinylpyrrolidone and magnesium stearate and compressed into tablet using appropriate tooling.

### EXAMPLE 4

| **Ingredients** | **Quantity (mg)** |
|---|---|
| **Intragranular** | |
| Valganciclovir hydrochloride (amorphous) eq. to 450 mg of Valganciclovir | 496.3 |
| Microcrystalline cellulose | 79.975 |
| Cross-linked polyvinylpyrrolidone | 21.0 |
| Polyvinylpyrrolidone | 14.0 |
| Magnesium stearate | 3.50 |
| **Extragranular** | |
| Microcrystalline cellulose | 79.975 |
| Magnesium stearate | 5.25 |
| **Total** | **700** |

### Procedure:

Valganciclovir hydrochloride (amorphous) was mixed with microcrystalline cellulose, cross-linked polyvinylpyrrolidone, polyvinylpyrrolidone and magnesium stearate and compacted with a roller compactor. The compacts were sized into granules by milling, mixed with microcrystalline cellulose and magnesium stearate and compressed into tablet using appropriate tooling.

### EXAMPLE 5

| **Ingredients** | **Quantity (mg)** |
|---|---|
| **Intragranular** | |
| Valganciclovir hydrochloride (amorphous) eq. to 450 mg of Valganciclovir | 496.3 |
| Microcrystalline cellulose | 79.975 |
| Cross-linked polyvinylpyrrolidone | 10.5 |
| Polyvinylpyrrolidone | 14.0 |
| Magnesium stearate | 3.50 |
| **Extragranular** | |
| Microcrystalline cellulose | 79.975 |
| Cross-linked polyvinylpyrrolidone | 10.5 |
| Magnesium stearate | 5.25 |
| **Total** | **700** |

### Procedure:

Valganciclovir hydrochloride (amorphous) was mixed with microcrystalline cellulose, cross-linked polyvinylpyrrolidone, polyvinylpyrrolidone and magnesium stearate and compacted with a roller compactor. The compacts were sized into granules by milling, mixed with microcrystalline cellulose, cross-linked polyvinylpyrrolidone and magnesium stearate and compressed into tablet using appropriate tooling.

## Claims

1. A dry process for the preparation of valganciclovir hydrochloride solid dosage forms wherein the process comprises mixing amorphous valganciclovir hydrochloride with one or more pharmaceutically acceptable excipient(s) and forming into a solid dosage form.

2. The process according to claim 1 wherein the process comprises compacting valganciclovir hydrochloride alone or mixed with one or more of pharmaceutically acceptable excipient(s) by roller compactor or slugging; sizing the compacts or slugs into granules by milling; optionally mixing the granules with one or more of pharmaceutically acceptable excipients and forming a solid dosage form.

3. The process according to claim 2 wherein the compaction is done by roller compactor.

4. The process according to claim 1 wherein the mixture is directly compressed into a tablet.

5. A solid dosage form comprising amorphous Valganciclovir hydrochloride, filler, disintegrant, binder and lubricant prepared by the process of claim 1.

6. A solid dosage form comprising amorphous valganciclovir hydrochloride, microcrystalline cellulose, cross-linked polyvinylpyrrolidone, polyvinylpyrrolidone and magnesium stearate prepared by the process of claim 1.

7. The solid dosage form according to claim 5 wherein the solid dosage form is a tablet.

8. The solid dosage form according to claim 5 wherein the solid dosage form is a capsule.

9. A solid dosage form according to claim 5 wherein it additionally comprises another drug in a therapeutically effective amount.

10. A pharmaceutical composition for the treatment of cytomegalovirus (CMV) retinitis in patients with acquired immunodeficiency syndrome (AIDS) and for the prevention of CMV disease in kidney, heart and for kidney-pancrease transplant patients, comprising a solid dosage form of amorphous valganciclovir hydrochloride as claimed in any of claims 5-9.

## Patentansprüche

1. Trockenes Verfahren zur Herstellung fester Dosierformen von Valganciclovir-Hydrochlorid, umfassend das Mischen von amorphem Valganciclovir-Hydrochlorid mit einem oder mehreren pharmazeutisch zulässigen Hilfsstoff(en) und das Formen in eine feste Dosierform.

2. Verfahren nach Anspruch 1, umfassend das Kompaktieren von Valganciclovir-Hydrochlorid allein oder gemischt mit einem oder mehreren pharmazeutisch zulässigen Hilfsstoff(en) mittels eines Walzenkompaktors oder das Trockengranulieren; Einstellen der Größe der Presskörper oder des Trockengranulats durch Mahlen zu Granalien; ggf. Mischen der Granalien mit einem oder mehreren pharmazeutisch zulässigen Hilfsstoff(en) und Formen in eine feste Dosierform.

3. Verfahren nach Anspruch 2, worin das Verdichten mittels eines Walzenkompaktors erfolgt.

4. Verfahren nach Anspruch 1, worin das Gemisch unmittelbar zu einer Tablette gepresst wird.

5. Feste Dosierform, hergestellt nach dem Verfahren gemäß Anspruch 1, umfassend amorphes Valganciclovir-Hydrochlorid, Füllstoff, Sprengmittel, Bindemittel und Schmiermittel.

6. Feste Dosierform, hergestellt nach dem Verfahren gemäß Anspruch 1, umfassend amorphes Valganciclovir-Hydrochlorid, mikrokristalline Zellulose, vernetztes Polyvinylpyrrolidon, Polyvinylpyrrolidon und Magnesiumstearat.

7. Feste Dosierform nach Anspruch 5, worin die feste Dosierform eine Tablette ist.

8. Feste Dosierform nach Anspruch 5, worin die feste Dosierform eine Kapsel ist.

9. Feste Dosierform nach Anspruch 5, ferner einen anderen Arzneistoff in therapeutisch wirksamer Menge umfassend.

10. Pharmazeutische Zusammensetzung zur Behandlung der Zytomegalie(CMV)-Retinitis bei Patienten mit erworbenem Immundefektsyndrom (AIDS) und zur Verhütung der Zytomegalie der Niere, des Herzens und für Patienten mit Nieren-Pankreas-Transplantation, umfassend eine feste Dosierform von amorphem Valganciclovir-Hydrochlorid nach einem der Ansprüche 5 bis 9.

## Revendications

1. Procédé pour la préparation par voie sèche de formes posologiques solides de chlorhydrate de valgancyclovir dans lequel le procédé comprend le mélange du chlorhydrate de valgancyclovir amorphe avec un ou plusieurs excipients pharmaceutiquement acceptables et la formation en une forme posologique solide.

2. Procédé selon la revendication 1, dans lequel le procédé comprend le compactage du chlorhydrate de valgancyclovir seul ou mélangé avec un ou plusieurs excipients pharmaceutiquement acceptables par un compacteur à rouleaux ou le briquetage ; le calibrage des éléments compactés ou des briquettes en granules par broyage ; éventuellement le mélange des granules avec un ou plusieurs des excipients pharmaceutiquement acceptables et la formation d'une forme posologique solide.

3. Procédé selon la revendication 1, dans lequel le compactage est fait par un compacteur à rouleaux.

4. Procédé selon la revendication 1, dans lequel le mélange est directement compressé en un comprimé.

5. Forme posologique solide comprenant du chlorhydrate de valgancyclovir amorphe, une charge, un délitant, un liant et un lubrifiant préparée par le procédé de la revendication 1.

6. Forme posologique solide comprenant du chlorhydrate de valgancyclovir amorphe, de la cellulose microcristalline, de la polyvinylpyrrolidone réticulée, de la polyvinylpyrrolidone et du stéarate de magnésium préparée par le procédé de la revendication 1.

7. Forme posologique solide selon la revendication 5, dans laquelle la forme posologique solide est un comprimé.

8. Forme posologique solide selon la revendication 5, dans laquelle la forme posologique solide est une capsule.

9. Forme posologique solide selon la revendication 5, dans laquelle elle comprend de manière additionnelle un autre médicament dans une quantité thérapeutiquement efficace.

10. Composition pharmaceutique pour le traitement de la rétinopathie à cytomégalovirus (CMV) chez des patients ayant le syndrome d'immunodéficience acquise (SIDA) et pour la prévention d'infection à CMV dans le rein, le coeur et pour des patients à greffe rein-pancréas, comprenant une forme posologique solide de chlorhydrate de valgancyclovir amorphe telle que revendiquée dans une quelconque des revendications 5-9.
